# EUROPEAN PATENT SPECIFICATION

(11) **EP 0 453 458 B1**
(45) Date of publication and mention of the grant of the patent: **19.04.1995**
(21) Application number: 90901579.4
(22) Date of filing: 08.01.1990
(51) Int. Cl.: C12N 15/87

(54) **PROCESS FOR PRODUCING A TRANSGENIC NON-HUMAN ANIMAL BY INTRODUCTION OF EXOGENOUS DNA IN SOMATIC AND GERM ANIMAL CELLS**
VERFAHREN ZUR HERSTELLUNG EINES TRANSGENEN; NICHT HUMANEN TIERES DURCH DAS EIN- BRINGEN VON EXOGENER DNA IN TIERZELLEN; DIE SOMATISCHEN UND KEIMBAHN URSPRUNG HABEN
PROCéDé DE PRODUCTION D'UN ANIMAL TRANSGENIC NON-HUMAIN DU A L'INTRODUCITON D'UN ADN EXOGèNE DANS DES CELLULES ANIMALES SOMATIQUES ET GERMINATIVES

(30) Priority: 10.01.1989 IT 1905089
(43) Date of publication of application: 30.10.1991
(73) Proprietor: CONSIGLIO NAZIONALE DELLE RICERCHE, 00185 Roma (IT)
(72) Inventor: SPADAFORA, Corrado, I-00165 Roma (IT)
(74) Representative: Gervasi, Gemma, Dr.
(86) International application number: EP9000032
(87) International publication number: WO9008192

(56) References cited:
- WO-A-87/05325
- proceedings of the national academy of sciences, volume 68, no 2, february 1971 US; B.G. Brackett et al.: "uptake of heterologous genome by mammalian spermatozoa and its transfer to ova through fertilization", pages 353-357.
- Cell, volume 57, 2 june 1989, Cell Press, M. Lavitrano et al.: "Sperm cells as vectors for introducing foreign DNA into Eggs: genetic transformation of mice", pages 717-723
- Cell, voluma 59, 20 october 1989, Cell Press., R.L. Brinster et al.:"No simple solution for making transgenic mice", pages 239-241.

## Description

### 1. Field of the invention

The present invention refers to a process for producing a transgenic non-human animal by introduction of exogenous DNA into somatic and germ animal cells.

In particular, the process consists in introducing DNA, exogenous or modified according to known techniques of recombinant DNA, into the spermatozoa head of the animal which is to be modified and in employing said modified spermatozoa for egg fertilization according to known techniques for artificial fertilization.

### 2.Prior technique

The creation of transgenic animals, that is of animals in which are permanently integrated genetic informations extraneous to their own genomes and deriving from other genetic systems, has been and still is an objective of primary importance for the study of genetic regulation, both for chemical and therapeutical ends and for breeding domestic mammals, fish, echinoderma and amphibia.

It is possible in fact to create animals with particular advantageous characteristics, such as e.g. rate of growth or resistance to certain diseases in the case of animals for breeding, or, viceversa, predisposition to certain diseases in the case of animals utilized for experimenting new drugs. The first attempts at obtaining transgenic animals go back to the middle of the seventies.

Those attempts were chiefly based on the manipulation of mice embryos or of cultured cells and on the direct DNA (e.g. SV 40) introduction into the mice blastocyst cavities, as described by M.H.L. & A. Mc Laren in Experimental Cell Research (1924) 86, 1-8, and by R. Jaenisch & B. Mintz (1974) in Proc. Natl. Acad. Sci. USA (1974) 71, 1250-54.

The results obtained were, however, disappointing, particularly because of the difficulty of the employed techniques.

In 1980 John W. Gordon et al. described in Proc. Natl. Acad. Sci USA (1980), 77 No. 12, 7380-84 a new technique for the genetic transformation of mice embryos by microinjection of cloned DNA into the pronucleus of fertilized oocytes.

The thus treated zygotes were re-implanted in the uterus of a pseudogravid mother and the gestation brought to term.

By means of this technique, which was refined in the last years, it was shown that it is possible to introduce exogenous DNA into mouse somatic and germ cells obtaining transgenic animals.

A typical example of transgenic animals obtained by the technique of the microinjection of exogenous DNA into fertilized eggs, and employed for experimenting anti-tumoral therapies, are the mice described in the U.S.P. 4.736.866, which show in their somatic and germ cells the presence of an oncogenic sequence.

The microinjection technique is being experimented in the field of breeding; howevwer, we do not have information of finally acquired results.

The DNA microinjection in fertilized oocytes is at any rate a complex and expensive technique, with low efficiency because of the high rate of abortions, the high rate of mosaicism in the obtained animals and the marked sterility of the same.

Another technique was suggested in 1987 (WO87/05325) using the ability of sperms of transferring substances bound to their surface into egg cells.

Accordingly genetic material, in form of nucleic acids, is either entrapped in artificial vesicles or processed with specific proteins and inorganic salts to form granulae. The so obtained envelopes, containing the genetic material, are then attached to the sperm surface and transferred with it into egg cells.

We have found no evidence about a real use of this technique, which in any case appears to be complex due to the number of materials to be used and operations to be performed.

### 3. The technical problem

The fundamental technical problem which is solved by the present invention is the introduction of DNA, treated according to known techniques of recombinant DNA, into the cellules of an animal pertaining to an animal species which does not actually posses the typical sequences of the introduced exogenous DNA, with the result that the genetic informations contained in said recombinant DNA is permanently integrated in the genomes of the treated individual and may therefore be transmitted to the successive progeny of the individual.

### D. Detailed description of the invention

The process for the introduction of cloned DNA into the cells of a different species according to the present invention is based on an experimental observation, namely the surprising easiness with which molecules, even if of large dimensions, succeed in penetrating into the spermatozoa head.

This property, typical of spermatozoa both of mammals and of other animal species, was utilized to modify the spermatozoa, introducing in them the cloned DNA to be transferred.

With the modified spermatozoa, the corresponding oocytes are then fertilized by means of the artificial fertilization techniques employed with unmodified spermatozoa.

According to a fundamental characteristic of the present invention, the spermatozoa of the species into which one wants to introduce the genetic informations extraneous to its own genomes, and originating from other systems, are utilized as vectors for transferring the extraneous DNA.

One of the possible ways of performing the process according to the present invention comprises the following steps:
a) preparation of an aqueous spermatozoa suspension
b) transformation of the spermatozoa with cloned DNA
c) "in vitro" fertilization of the oocytes by means of the modified spermatozoa
d) implantation of the fertilized oocytes into pseudogravid females of the selected species.

According to another possible way of performing the present invention, the modified spermatozoa may be employed directly for the animal fertilization without going through the "in vitro" oocyte fertilization and successive implantation of the same into pseudogravid females.

This form of realization of the invention is particularly useful in case the process is employed for breeding.

The process according to the invention is much simpler, much more rapid and less costly than the microinjection technique, and does not bring about abortions which are very frequent in the microinjection technique.

The invention will now be described in detail with particular reference to the modification of mice spermatozoa.

We have employed this animal because all the laboratory technique for its "in vitro" fertilization and for the study of the integration and expression of its genes are amply reported in the scientific literature.

As exogenous DNA we used p SV2 CAT, Polyoma and the human growth gene, because their restriction maps are described in literature and comprise base sequences which are not naturally present in mouse genome.

The identification of these sequences in the "positive" mouse, that is in the mouse obtained from the egg fertilized with the treated spermatozoa, allows to ascertain without the shadow of a doubt that the cloned DNA was actually introduced into the head of the treated spermatozoa and through these into the fertilized eggs and therefore integrated into the genome of the resulting transgenic individuals.

### a) preparations of the spermatozoa.

A spermatozoa suspension was prepared by pressing the epididymis of a male mouse into 1 ml PM buffer (prepared as described by D.G. Whittingam. Culture of Mouse - ove - (1971) - J Reprod. Fert. Supp. 14, p.7-21).

The spermatozoa suspension was centrifuged so to separate the spermatozoa which were again suspendend in 1 ml of buffer.

The above treatment was repeated 5 times so to "wash" the spermatozoa by assuring the complete elimination of seminal liquor. The buffer was modified eliminating sodium lactate, penicillin and steptomycin, substituting monosodiumphosphate by 0.15 mM bisodiumphosphate and changing the amount of sodium chloride to 120 mM. The spermatozoa suspension was then diluted to a concentration of 1-2 million/ml and incubated for a period of from 30 minutes to 3 hrs at a temperature of from 20° to 37°C, in air containing more than 5% to 10% of carbon dioxide.

### b) DNA transformation of the spermatozoa

To the diluted spermatozoa suspension, in fractions of 500 »l, is added the solution of the cloned, circular DNA which is intended to be inserted into the spermatozoa, and the mixture is further incubated for at least 30 minutes at a temperature of from 0° to 37°C, in air containing more than 5% to 10% of carbon dioxide.

At the lower temperatures, the insertion of DNA into the spermatozoa is easier, due to a reduced activity of endogenous nuclease of spermatozoa, but the incubation time is longer than 30 minutes.

The cloned circular DNA solution is added in an amount such as to obtain a final concentration into the cloned DNA mixture comprised between 0.4 and 2 »g/ml.

### c) egg fertilization

Mature mouse females are induced to superovulate by means of human choronic gonadotropin and the eggs are extracetd from the oviduct 14.5 hours after the injection (according to the method described by B. Hogan et al. in "Manipulating the Mouse Embryo A Laboratory Manual" CSM New York, 1986).

The eggs extracted from the oviducts are introduced into the 500 »l fractions of the transformed spermatozoa and incubated from 5 to 10 hours at a temperature of from 20° to 37°C, in air containing more than 5% to 10% of carbon dioxide. At the end of said period, the eggs are washed with M16 buffer (prepared as described by Whittingam -see point a) supra) and left for an entire night in 50 »l of the same buffer.

After 24 hours the embryos are surgically transferred, at the stage of two cellules, into the oviducts of pseudogravid females.

The offsprings deriving from these implants, at the age of three weeks, are amputated of a terminal tail fragment, from which the DNA is extracted which is analyzed with the aid of the "Southern blot" described in the book "Molecular Cloning": A Laboratory Manual" by T. Maniatis et al. - C.S.M., New York 1984.

This analysis allows to identify "positive" individuals, that is those whose genome posses, integrated or in episomic form, one or more copies of the same cloned DNA introduced into the starting spermatozoa.

The yield of "positive"individuals obtained following the process of the present invention is always higher than 30% up to 70%, and, what is more, no sterile individuals are found among them.

The successive genetic characterization of the positive animals is carried out with the two analysis methods of restriction and sequence.

The analysis of the genome DNA of positive mice was carried out according to two methods:

### Restriction Analysis

DNA restriction analysis by means of specific restriction enzymes which allow the subdivision of genome DNA into fragments. The obtained fragments are then fractionated by electrophoresis and transferred onto a nitrocellulose filter according to the known Southern blot technique. The filter is then treated with the probe specific for the initial cloned DNA employed for transforming the spermatozoa, made radioactive with P³² and exposed on a x-ray sensitive film.

The film will be exposed in correspondence with the sites where the radioactive probe was bound to the filter, that is in correspondence with each radioactive DNA bond, leaving a signal for each DNA fragment.

The presence of one or more signals, their number and the dimensions of the DNA fragments which they represent allow to conclude that sequences exist which are analogous to the positive mouse genome probe, and to determine a restriction map.

The analogy between this map and the one of the cloned DNA introduced into the spermatozoa from which the "positive" mouse originates proves that the original clone sequences are integrated into the transgenic mouse genome.

### - Sequence analysis

To analize the sequence of the bases constituing the "positive" mouse genome, equal amounts (15 »g) of genome DNA of a positive mouse and of the pUC13 plasmid resistant to ampicillin were restricted with the restriction EcoRI enzyme.

The two restricted DNA were then mixed, recircularized and introduced into Eschericia Coli HB101 bacteria, which were then cultivated on Agar + Ampicillin.

The positive colonies (that is the ones containing a cloned fragment) were separated, amplified and purified. The cloned fragment was then separated from the pUC13 vector by restriction with EcoRI and 626 bases were sequened from it using the Langer method.

It was thus possible to ascertain that the initial clone was transferred from the spermatozoa into the fertilized egg and then integrated into the genoma of the resulting individual.

Beside the two methods reported above, we have carried out an anlysis of the spermatozoa after their transformation with the cloned DNA, to the end of ascertaining the location of the exogenous DNA.

To this end we employed H³ labeled DNA, and various aliquots of the spermatozoa solutions after their incubation with labeled DNA were radio-autographed at the optical and at the electronic microscope.

The obtained results have evidenced that cloned DNA is specifically located inside the spermatozoa head in sub-equatorial position.

Traces of radioactivity in other regions of the spermatozoa are insignificant.

As it is known that the acrosomal fusion reaction between spermatozoa and oocyte at the moment of fertilization (with transferral of genetic material from spermatozoa to the egg) takes place in that particular region, it is believed that also the exogenous DNA is trasferred at the same time and in the same way as the DNA proper of the species. This mechanism might explain the surprising efficiency of the process according to the invention.

## Claims

1. Process for producing a transgenic non-human animal whose germ and somatic cells contain a sequence of exogenous DNA, said animal being able to reproduce fertile offsprings, characterized in that oocytes are fertilized by means of spermatozoa of animal which one intends to modify, said spermatozoa being modified by the introduction into the spermatozoa head of exogenous DNA and then employed for oocytes fertilization, the embryos being transferred into recipient animal directly and non-surgically.

2. Process for producing a transgenic non-human animal whose germ and somatic cells contain a sequence of exogenous DNA. said animal being able to reproduce fertile offsprings characterized in that spermatozoa of the animal which one intends to modify are modified by the introduction into the spermatozoa head of exogeneous DNA and then said modified spermatozoa are directly employed for animal artificial fertilization without going through the "in vitro" oocytes fertilization and without either surgical or non-surgical implantation of the fertilized oocytes or embryos.

3. Process according to claims 1 and 2 wherein said spermatozoa are collected, an aqueous spermatozoa suspension is prepared by means of a buffer solution, said suspension is incubated with cloned DNA and the obtained modified spermatozoa are used for the oocyte fertilization.

4. Process according to claims 1 and 2, characterized in that said aqueous spermatozoa suspension is buffered with a FM buffer, diluted to a spermatozoa concentration of 1-2 millions/ml and incubated at 20°C to 37°C for a period of from 30 minutes to 3 hours, in air containing more than 5% up to 10% of carbon dioxide.

5. Process according to claims 1 and 2, characterized in that the cloned DNA solution to be inserted into the spermatozoa is added to said incubated aqueous spermatozoa suspension and incubated further for at least 30 minutes at a temperature of from 0°C to 37°C, said solution being added in an amount such as to have a final cloned DNA concentration in the mixture of from 0.4 to 2 »g/ml.

6. Process according to claim 1, characterized in that the oocytes to be fertilized are added to the aqueous incubated spermatozoa suspension and the mixture is incubated for 5 to 10 hours at a temperature of from 20°C to 37°C, in air containing more that 5% up to 10% of carbon dioxide.

## Patentansprüche

1. Verfahren zur Erzeugung eines transgenen, nicht humanen Tieres, dessen Keimbahn- und somatischen Zellen eine Sequenz von exogener DNA enthalten, wobei das genannte Tier fähig ist, fruchtbare Nachkommen zu reproduzieren, dadurch gekennzeichnet, daß Oocyten mittels Spermatozoen eines Tieres, das modifiziert werden soll, befruchtet werden, wobei die genannten Spermatozoen durch Einführen exogener DNA in den Spermatozoenkopf modifiziert und dann für die Befruchtung von Oocyten verwendet werden, und die Embryonen in das Empfängertier direkt und nicht chirurgisch überführt werden.

2. Verfahren zur Erzeugung eines transgenen, nicht humanen Tieres, dessen Keimbahn- und somatischen Zellen eine Sequenz von exogener DNA enthalten, wobei das genannte Tier fähig ist, fruchtbare Nachkommen zu reproduzieren, dadurch gekennzeichnet, daß die Spermatozoen des Tieres, welches modifiziert werden soll, durch Einführung von exogener DNA in den Spermatozoenkopf modifiziert werden und dann die genannten modifizierten Spermatozoen direkt für die künstliche Befruchtung des Tieres verwendet werden, ohne die "in vitro" Oocytenbefruchtung zu durchlaufen und ohne chirurgische oder nicht chirurgische Implantation der befruchteten Oocyten oder Embryonen.

3. Verfahren gemäß den Ansprüchen 1 und 2, worin die genannten Spermatozoen gesammelt werden, eine wässrige Spermatozoensuspension mittels einer Pufferlösung hergestellt wird, die genannte Suspension mit klonierter DNA inkubiert wird und die erhaltenen modifizierten Spermatozoen für die Oocytenbefruchtung verwendet werden.

4. Verfahren gemäß den Ansprüchen 1 und 2, dadurch gekennzeichnet, daß die genannte Spermatozoensuspension mit einem FM-Puffer gepuffert, auf eine Spermatozoenkonzentration von 1-2 Millionen/ml verdünnt und bei 20°C bis 37°C über einen Zeitraum von 30 Minuten bis 3 Stunden in Luft, die mehr als 5% bis 10% Kohlendioxid enthält, inkubiert wird.

5. Verfahren gemäß den Ansprüchen 1 und 2, dadurch gekennzeichnet, daß die Lösung der klonierten DNA, die in die Spermatozoen insertiert werden soll, zu der genannten inkubierten wässrigen Spermatozoensuspension zugegeben wird und für mindestens 30 Minuten bei einer Temperatur von 0°C bis 37°C weiter inkubiert wird, wobei die Lösung in einer solchen Menge zugegeben wird, daß die endgültige Konzentration der klonierten DNA in der Mischung von 0,4 bis 2 »g/ml beträgt.

6. Verfahren gemäß Anspruch 1, dadurch gekennzeichnet, daß die Oocyten, die befruchtet werden sollen, zu der wässrigen inkubierten Spermatozoensuspension gegeben werden und die Mischung 5 bis 10 Stunden bei einer Temperatur von 20°C bis 37°C in Luft, die mehr als 5% bis 10% Kohlendioxid enthält, inkubiert wird.

## Revendications

1. Procédé de production d'un animal transgénique non humain dont les cellules germinatives et somatiques contiennent une séquence d'ADN exogène, ledit animal étant capable de produire une descendance fertile, caractérisé en ce que les ovocytes sont fertilisés au moyen de spermatozoïdes de l'animal que l'on veut modifier, lesdits spermatozoïdes étant modifiés par introduction d'ADN exogène dans la tête du spermatozoïde, et ensuite employés pour la fertilisation des ovocytes, les embryons étant transférés dans l'animal receveur directement et par voie non chirurgicale.

2. Procédé de production d'un animal transgénique non humain dont les cellules germinatives et somatiques contiennent une séquence d'ADN exogène, ledit animal étant capable de produire une descendance fertile, caractérisé en ce que les spermatozoïdes de l'animal que l'on veut modifier sont modifiés par introduction d'ADN exogène dans la tête du spermatozoïde, et ensuite les spermatozoïdes modifiés sont directement employés pour la fertilisation artificielle de l'animal, sans passer par la fertilisation in vitro des ovocytes et sans implantation chirurgicale ni implantation non chirurgicale des ovocytes fertilisés ou des embryons.

3. Procédé selon les revendications 1 et 2, dans lequel lesdits spermatozoïdes sont recueillis, une suspension aqueuse des spermatozoïdes est préparée au moyen d'une solution tampon, ladite suspension est incubée avec l'ADN cloné et les spermatozoïdes modifiés obtenus sont utilisés pour la fertilisation des ovocytes.

4. Procédé selon les revendications 1 et 2, caractérisé en ce que ladite suspension aqueuse de spermatozoïdes est tamponnée avec un tampon FM, diluée jusqu'à une concentration en spermatozoïdes de 1-2 millions/ml et incubée à 20 - 37°C pendant une durée de 30 minutes à 3 heures, dans de l'air contenant plus de 5% et jusqu'à 10% de dioxyde de carbone.

5. Procédé selon les revendications 1 et 2, caractérisé en ce que la solution d'ADN cloné à insérer dans les spermatozoïdes est ajoutée à ladite suspension aqueuse incubée de spermatozoïdes, et encore incubée pendant au moins 30 minutes à une température de 0°C à 37°C, ladite solution étant ajoutée en une quantité suffisante pour obtenir une concentration finale en ADN cloné dans le mélange de 0,4 à 2 »g/ml.

6. Procédé selon la revendication 1, caractérisé en ce que les ovocytes à fertiliser sont ajoutés à la suspension aqueuse incubée de spermatozoïdes et le mélange est incubé pendant 5 à 10 heures à une température de 20°C à 37°C, dans de l'air contenant plus de 5% et jusqu'à 10% de dioxyde de carbone.
